(19) Europäisches Patentamt
European Patent Office
Office européen des brevets

(11) Publication number: **0 506 977 A1**

(12) # EUROPEAN PATENT APPLICATION published in accordance with Art. 158(3) EPC

(21) Application number: **91918898.7**

(22) Date of filing: **24.10.91**

(86) International application number: **PCT/JP91/01452**

(87) International publication number: **WO 92/07960 (14.05.92 92/11)**

(51) Int. Cl.5: **C12Q 1/70**, C12Q 1/68, //C12N15/51,C12N15/11, (C12Q1/70,C12R1:92)

(30) Priority: **24.10.90 JP 284465/90**

(43) Date of publication of application: **07.10.92 Bulletin 92/41**

(84) Designated Contracting States: **AT BE CH DE DK ES FR GB GR IT LI LU NL SE**

(71) Applicant: **Chugai Pharmaceutical Co., Ltd.**
**5-1, Ukima 5-chome Kita-ku**
**Tokyo 115(JP)**
Applicant: **Arima, Terukatsu**
**5-1-403, Hirano-cho**
**Kagoshima-shi, Kagoshima 892(JP)**

(72) Inventor: **ARIMA, Terukatsu**
**5-1-403, Hirano-cho**
**Kagoshima-shi, Kagoshima 892(JP)**
Inventor: **YAMAMOTO, Osami**
**3-30-304, Izumi-cho**
**Numazu-shi, Shizuoka 410(JP)**
Inventor: **TSUCHIYA, Masayuki, Chugai Pharmac. Co., Ltd.**
**Fujigotemba Kenkyusyo, 1-135, Komakado**
**Gotenba-shi, Shizuoka 412(JP)**
Inventor: **OSHIMA, Masanobu**
**Chugai-Gotenbaryo, 1512, Higashitanaka**
**Gotenba-shi, Shizuoka 412(JP)**
Inventor: **YAGASAKI, Mitsuro**
**4-33-2-202, Horie**
**Urayasu-shi, Chiba 279(JP)**
Inventor: **MATSUOKA, Yukio**
**C-411, 4-3, Inumakata**
**Urawa-shi, Saitama 336(JP)**

(74) Representative: **Vossius & Partner**
**Siebertstrasse 4 P.O. Box 86 07 67**
**W-8000 München 86(DE)**

(54) **METHOD OF DETECTING NON-A NON-B HEPATITIS SPECIFIC GENE AND REAGENT COMPOSITION USED THEREFOR.**

(57) A method of detecting blood-transmissible non-A non-B hepatitis specific gene either by carrying our DNA-DNA, DNA-RNA or RNA-RNA hybridization by using a probe comprising a cDNA or a part thereof inserted into any of the specified phage clones (listed in Table 1 of the specification) prepared by inserting a cDNA coding for a blood-transmissible non-A non-B hepatitis specific gene protein into a phage, or by carrying out the growth of a nucleic acid sequence by using a primer comprising a part of said cDNA; and a reagent for use in detecting blood-transmissible non-A non-B hepatitis specific gene, comprising said probe and/or said primer.

EP 0 506 977 A1

Fig.2(1)

```
                10         20         30         40         50         60         70         80         90        100
#2232  GAATTCTTCA CAGAGTTGGA TGGGGTGCGG CTGCACAGGT ACGCTCCGGC GTGCAAACCT CTCCTGCGGG ATGAGGTCAC GTTCCAGGTC GGGCTCAACC
#2230  ---------- ---------- ---------- ---------- ---------- ---------- ---------- ---------- ---------- ----------
#2206  ---------- ---------- C--------- ---------- ---------- -----G---- ---------- ---------- A--------- ----------
#2258  ---------- ---------- C--------- ---------- ---------- -----G---- ---------- ---------- A--------- ----------
#2248  ---------- ---------- ---------- T--------- ---------- ---------- -----A---- ---------- A--T------ ----------
#2220  ---------- -----C---- ---------- T--------- ---------- ------G--- -----A---- ---------- A--T------ ----------
#2216  ---------- ---------- ---------- T--------- ---------- TGCA--G--- -----A---- ---------- A--T------ ----------
#2246  ---------- -G-------- ------A--- ---------- ---------- ------G--A -----A---- ---------- A--------- ----------
#2211  ---------- -G--A----- ---------- --A------- ---------- ------G--- -----A---- ---------- A--------- ----------

               110        120        130        140        150        160        170        180        190        200
#2232  AATACCCGGT TGGATCACAG CTCCCATGCG AGCCCGAACC GGATGTGGCG GTGCTCACTT CCATGCTCAC CGACCCCACC CACATTACAG CAGAAGCGGC
#2230  ---------- ---------- ---------- ---------- ---------- ---------- ---------- ---------- ---------- ----------
#2206  ---------- ---G----C- --------T- ---------- ------AA-A ---G------ ---------- -------T-- ---------- -G---A-T--
#2258  ---------- ---G------ --------T- ---------- ------AA-A ---G------ ---------- -------T-- ---------- -G---A-T--
#2248  ---T------ ---G------ --------T- ---------- ------AA-A ---A----C- ---------- -------T-- ---------- ----GA----
#2220  ---T------ ---G------ -----G--T- ---------- ------AA-- ---A------ ---------- -------T-- ---------- ----GA----
#2216  ---T------ ---G------ --------T- ---------- ------AA-A ---A----C- ---------- -------T-- ---------- ----G-----
#2246  ---TT--A-- ---------- --------T- -------G-- ------A--- ---------- ---------- -------T-- ---------- ----GA----
#2211  ---T------ ---G------ --------T- -A-------- ------AAT- ---G----C- -T-------- -------T-- --T------- ----GA----

               210        220        230        240        250        260        270        280        290        300
#2232  TAGGCGCAGG CTGGCCAGAG GGTCTCCTCC TTCCTTGGCC AGCTCTTCAG CTAGTCAGTT GTCTGCGCCC TCCTTGAAGG CGACATGCAC TACCCATCAT
#2230  ---------- ---------- ---------- ---------- ---------- ---------- ---------- ---------- ---------- ----------
#2206  C-----T--- T-------G- --AG---C-- ---------- ---------- ---------- ------A--T ---------- -------T-- ----------
#2258  C-----T--- T-------G- --AG---C-- ---------- ---------- ---------- ------A--T ---------- -------T-- ----------
#2248  -G----T--- ---------- -------C-- ---------- --------G- ---------- ---------T ---------- ---------- -----G----
#2220  -G----T--- ---------- -------C-- ---------- --------G- ---------- ---------- ---------- -A-------- -----G----
#2216  -G----T--- ---------- -------C-- ---------- --------G- ---------- ---------- *---*----- *--------- -----G----
#2246  --A---T--- --------G- ----C--C-- C--------- ---------- ---------- ---------- --T------- ---------- -----C----
#2211  --A---T--- ---------- -------C-- ---T------ ---------- ---------- ---------- ---------- ---------- C----G----
```

The present invention relates to a method of detecting genes specific to blood-borne non-A, non-B hepatitis and agents for the detection. Particularly, it relates to a method of detecting a blood-borne non-A, non-B hepatitis-specific gene, and agents for such a detection, characterized by using a DNA sequence coding for a blood-borne non-A, non-B hepatitis-specific antigen protein or a part thereof.

Among viral hepatitis, the A-type and the B-type viruses therefor have been found, immunological diagnoses thereof have become possible, and vaccines against these viruses have been developed. At present, however, for the non-A, non-B type hepatitis, which is neither an A-type nor a B-type, the true nature has not been clarified because the amount of the pathogenic virus in an organism is very small. The blood-borne non-A, non-B hepatitis comprises at least 90% of hepatitis occurring after a blood transfusion, and 10 to 20% of patients having received a blood transfusion suffer this hepatitis (Experimental Medicine Vol. 7, 196 - 201 (1989)).

Scientists have searched for an antigen protein related to blood-borne non-A, non-B hepatitis, using the blood of patients. For example, Choo et al. (Science, 244. 359 - 362 (1989)) and EP 0318 216 A1 reported that a part of a non-A, non-B hepatitis virus gene was isolated, by transferring and infecting blood from a blood-borne non-A, non-B patient to a chimpanzee, extracting RNA from the serum, and preparing a cDNA library from the RNA. It was reported that, as a result of testing blood samples of Japanese, using an immunological diagnostic agent prepared on the basis of the above viral gene fragments, blood samples infected with non-A, non-B hepatitis virus were diagnosed at a ratio of 60 to 70%.

Japanese Unexamined Patent Publication No. 64-2576, on the other hand, discloses that the blood of a non-A non-B hepatitis patient was transferred to a chimpanzee, RNA was extracted from hepatic cells thereof, and the RNA was used to clone a gene for a non-A, non-B hepatitis-specific antigen protein.

In the former publication, the obtained findings are most insufficient, considering that the diagnostic ratio of the diagnostic agent for non-A, non-B hepatitis virus-infected blood samples is low, the number of nucleotides of the cloned gene is too small in comparison with the expected number of nucleotides of a blood-borne non-A, non-B hepatitis virus gene, and as stated by the author in this article, there is a possibility that two or more kinds of blood-borne non-A, non-B hepatitis viruses are present.

In the latter publication, no direct evidence is given showing that the cloned gene is specific to human non-A, non-B hepatitis.

As in both of the above cases the RNA obtained was from a chimpanzee and the RNA was not directly extracted from a human patient suffering human blood-borne non-A, non-B hepatitis, it is not clear whether the cloned DNA correctly reflects a gene for a human non-A, non-B hepatitis virus. Therefore, a new approach is necessary when it is considered that the development of diagnostic agents having a high accuracy and vaccines is urgently required.

As one approach, a coinventor of this invention has directly extracted RNA from blood of non-A, non-B hepatitis patients, and reported a result thereof using a method described hereinafter by the inventor (Experimental Medicine Vol. 7, 196 - 201 (1989)).

The present inventors, as a result of intensive research, succeeded in directly extracting RNA from hepatic cells or blood of non-A, non-B hepatitis patients, cloning DNA coding for blood-borne non-A, non-B hepatitis-specific antigen protein on the basis of the RNA, and expressing the DNA in E.coli. Non-A, non-B hepatitis-specific antigenic proteins thus obtained are a useful agent for the diagnosis of non-A, non-B hepatitis by measuring antibodies to the antigenic proteins.

Note, in the B-type hepatitis, the phase at which antibodies to viral antigenic proteins occur, or an amount thereof, is different depending on the condition of the patient and the kind of antigenic proteins. Generally, it is considered that, at an initial phase of the infection antibodies do not occur, after about 3 months antibodies to HBc (B-type hepatitis virus core protein) and HBe (B-type hepatitis virus envelop protein) occur, and after several more months HBs (B-type hepatitis virus surface antigen) occurs (ed. H. Suzuki, Kan-en (Hepatitis), Nan-un do, 1989).

With regard to non-A, non-B hepatitis, there is a report that the occurrence of a particular antibody does not necessarily conform to the presence of a corresponding antigen gene (A.J. Weiner et al., Lancet, 335, page 1 (1990); J.A. Garson et al., Lancet, 335, 1419 (1990).

Considering the above, there is a need to develop a reliable diagnostic method by directly confirming the presence of non-A, non-B hepotitis virus.

The present inventors found that the detection of a non-A, non-B hepatitis-specific gene is possible by using probes and/or primers on the basis of nucleotide sequences of DNA clones succesfully cloned by the present inventors as a means for direct determination of the presence of non-A, non-B hepatitis-specific gene, and accomplished the present invention on that basis.

Namely, in a method of detecting a non-A, non-B hepatitis-specific gene by a specific gene amplification using primers and hybridization (double strand formation) using probes, the present invention relates to a method of detecting a non-A, non-B hepatitis-specific gene and agents therefor, characterized by using, as probes and/or primers a cDNA nucleotide sequence inserted into one of the phage clones shown in Table 1, or a part thereof.

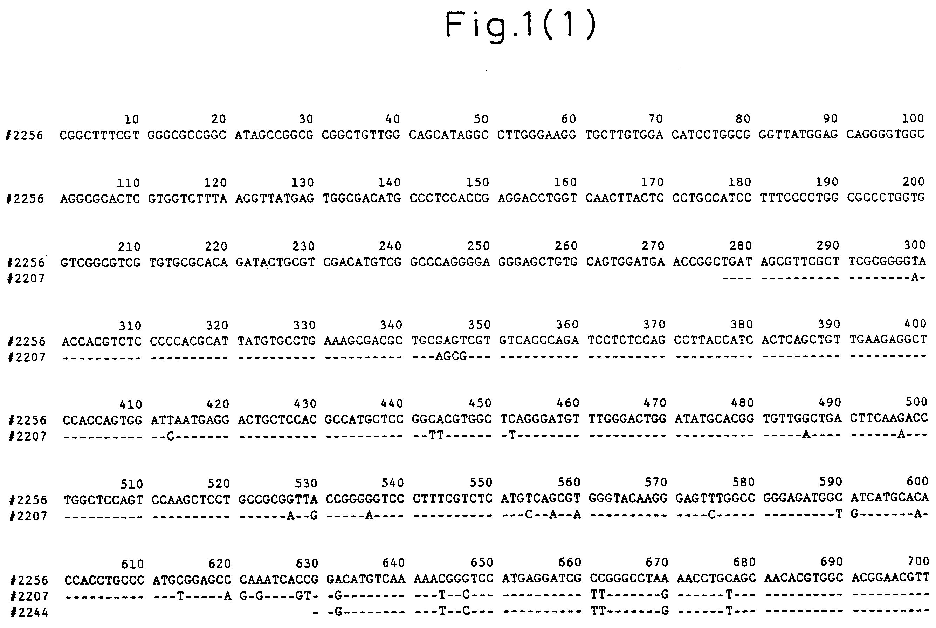

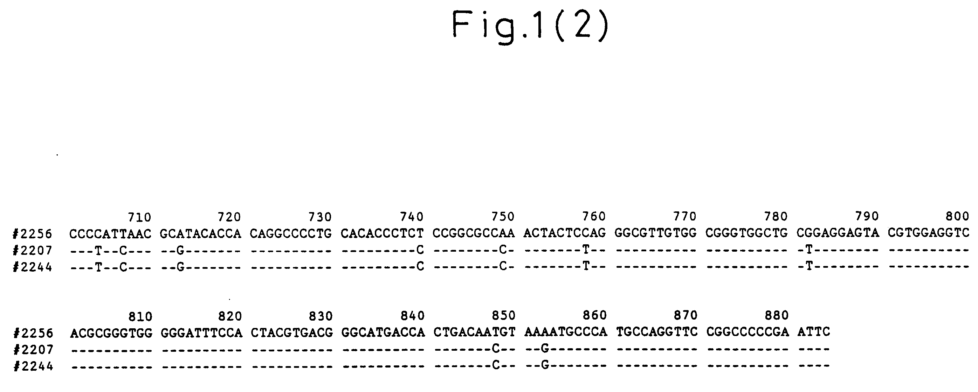

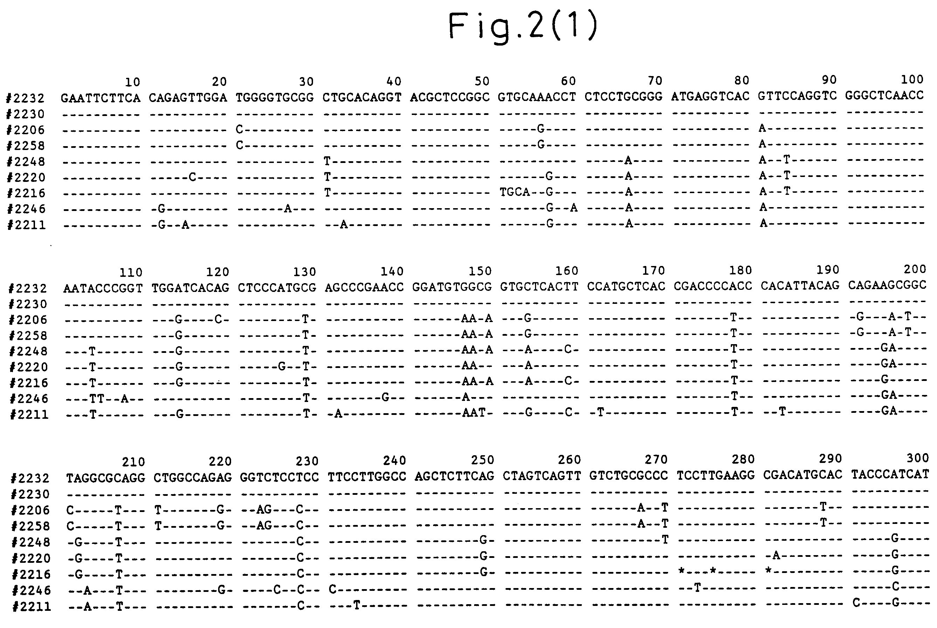

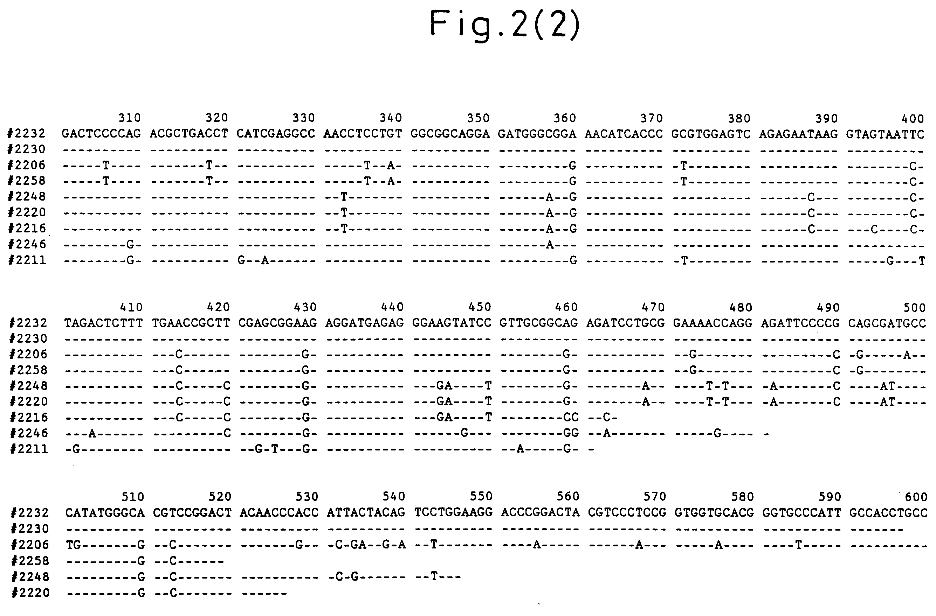

Figures 1 and 2 represent the homology of inserts of nucleotide sequences of a phage vector λHC 2256, 2207, 2244, 2232, 2230, 2206, 2258, 2248, 2220, 2246, 2211 and 2216.

The present detection method of detecting a non-A, non-B hepatitis-specific gene using probes and/or primers is based on hybridization (duplex formation) of nucleic acid molecules (Maniatis T. et al. Molecular Cloning Laboratory Manual, Cold Spring Harbor Press).

According to this method, upon the introduction of a foreign nucleotide chain (probe) into a denatured nucleic acid sample, the foreign probe binds to a complementary portion of the denatured nucleic acid to thus form a duplex. The principle of this reaction is the same when the nucleic acid is RNA or DNA. Nevertheless, the stability of the resulting duplex varies to some extent, depending on the complementarity of nucleic acid sequences in the duplex. Therefore, to efficiently carry out a detection of a non-A, non-B hepatitis-specific gene, it is important to use a probe and/or primer which form a duplex highly specific to a nucleic acid sample to be tested.

Although the conditions for forming a duplex are described in detail in references (J. Sambrook et al., Molecular Cloning 9.47, 11.45, Cold Spring Harbor Laboratory Press (1989)), in any case when the probe and/or primer used are specific to a nucleotide sequence of a nucleic acid sequence to be tested, and have a sufficient length, a specific and stable duplex is formed separately from other related nucleic acid sequences, and the presence of a specific nucleic acid sequence in a given nucleic acid sample can be determined by, for example, measuring a label substance (coloring agent, chemical luminescent substance or radioactive substance) attached to the probe.

It is considered that a non-A, non-B hepatitis virus or a specific nucleic acid is present in only a very small amount in the blood and tissues, and where such a very small amount of nucleic acids should be identified, it is difficult to directly detect the same by a probe as described above. Therefore, in such a case, after amplifying a target nucleic acid using primers comprising a specific sequence, the target nucleic acid can be more sensitively identified by forming a duplex with a probe. Alternatively, after sufficiently amplifying a target nucleic acid using primers as described above, the target nucleic acid can be identified by staining the nucleic acid with e.g. ethidium bromide. Several methods of gene amplification are known (Experimental Medicine, Vol. 8, No. 9 (1990), Yodo-sha, PCR and Application thereof), and such gene amplification methods can be applied.

The present inventors, as stated above, succeeded in isolating 61 DNA clones coding for a non-A, non-B hepatitis-specific antigenic protein, on the basis of hepatic cells and blood samples from hepatic carcinonoma patients suffering from non-A, non-B hepatitis. These DNA clones, as shown in Table 1, were inserted into a phage to thereby form recombinant phages λHC, which have been deposited to the Microorganism Deposition Center, Fermentation Research Institute, Agency of Industrial Science and Technology, and some also have been deposited as an international deposition under the Budapest Treaty.

Table 1

| Phage clone No. | Number of domestic deposition (FERM P-) | Number of international deposition (FERM BP-) |
|---|---|---|
| λHC 432 | 10897 | |
| λHC 436 | 10898 | |
| λHC 512 | 10841 | 2951 |
| λHC 522 | 10842 | |
| λHC 524 | 10843 | |
| λHC 526 | 10844 | |
| λHC 2206 | 10845 | 2952 |
| λHC 2207 | 10846 | 2953 |
| λHC 2211 | 10876 | 2956 |
| λHC 2216 | 10877 | 2957 |
| λHC 2217 | 10852 | |
| λHC 2220 | 10853 | 2954 |
| λHC 2225 | 10854 | |
| λHC 2404C | 10899 | |
| λHC 2405B | 10900 | |
| λHC 2410A | 10905 | |
| λHC 2410C | 10918 | 2967 |
| λHC 2410D | 10934 | |
| λHC 2413 | 10919 | |
| λHC 2414A | 10906 | |
| λHC 2424A | 10911 | |

Table 1 (continued)

| Phage clone No. | Number of domestic deposition (FERM P-) | Number of international deposition (FERM BP-) |
|---|---|---|
| λHC 2501 | 10920 | |
| λHC 2502 | 10847 | |
| λHC 2505 | 10921 | |
| λHC 2507 | 10935 | |
| λHC 2508 | 10859 | |
| λHC 2509 | 10936 | |
| λHC 2512 | 10882 | |
| λHC 2514 | 10860 | |
| λHC 2516 | 10861 | |
| λHC 2533 | 10907 | 2963 |
| λHC 2534 | 10937 | |
| λHC 2535 | 10883 | |
| λHC 2602 | 10908 | |
| λHC 2603B | 10922 | |
| λHC 2607 | 10909 | 2964 |
| λHC 2230 | 10916 | 2966 |
| λHC 2232 | 10930 | 2968 |
| λHC 2239 | 10931 | |
| λHC 2240 | 10855 | |
| λHC 2241 | 10856 | |
| λHC 2242 | 10857 | |

Table 1 (continued)

| Phage clone No. | Number of domestic deposition (FERM P-) | Number of international deposition (FERM BP-) |
|---|---|---|
| λHC 2243 | 10878 | |
| λHC 2244 | 10879 | 2958 |
| λHC 2246 | 10858 | 2955 |
| λHC 2248 | 10880 | 2959 |
| λHC 2249 | 10917 | |
| λHC 2250 | 10904 | |
| λHC 2252 | 10881 | |
| λHC 2255 | 10889 | |
| λHC 2256 | 10890 | 2960 |
| λHC 2258 | 10891 | 2961 |
| λHC 2259 | 10892 | |
| λHC 2263 | 10893 | |
| λHC 2264 | 10932 | |
| λHC 2265 | 10933 | |
| λHC 2268 | 10894 | |
| λHC 2270 | 10895 | 2962 |
| λHC 2271 | 10896 | |
| λHC 2608 | 10923 | |
| λHC 2610 | 10910 | 2965 |

Since the serological reaction of a protein encoded by a cDNA contained in each clone is highly specific to non-A, non-B hepatitis, it is believed that the DNA sequences contained in these clones are a part of the non-A, non-B hepatitis virus, or a nucleic acid closely related thereto. Accordingly, DNA sequences contained in clones shown in Table 1, or a part thereof, or RNAs corresponding thereto, may be used as highly specific probes in a method of detecting a non-A, non-B hepatitis-specific gene based on DNA-DNA, DNA-RNA or RNA-RNA hybridization. Moreover, a part of a DNA sequence contained in each clone shown in Table 1, and a complementary sequence thereof, may be used as a primer in a gene amplification process.

Among these phage clones, the nucleotide sequences of cDNA inserts of 18 strains deposited as an international deposition are shown in SEQ ID NOs: 1 to 18. Figs. 1 and 2 show an array of nucleotide sequences of cDNAs having a high homology, arrayed by comparing the homology therebetween. From such a comparison of nucleotide sequences, it is clear that these clones encode a limited region. For example, clone λHC 2256, λHC 2207 and λHC 2244 partially overlap; and λHC 2232, λHC 2230, λHC 2206, λHC 2258, λHC 2248, λHC 2220, λHC 2216, λHC 2246 and λHC 2211 almost overlap. Nevertheless, in portions wherein nucleotide sequences of these clones correspond to each other, their nucleotide sequences are not always identical, and there are substantial mutations. Therefore, as a probe for detecting a non-A, non-B hepatitis-specific gene or primers for an amplification of a non-A, non-B hepatitis-specific gene, a probe or primers which can hybridize with various mutants are preferably used. For this purpose, it is sufficient to use nucleotide portions involving less mutations among the above-mentioned sequences.

To select such a portion of a nucleotide sequence, for example, in a region wherein two corresponding nucleotide sequences are shown, two nucleotide sequences are arbitrarily selected and the homology between the nucleotide sequences is tested, and thereafter, regions wherein the homology between any pair of the above-mentioned nucleotide sequences shows at least a predetermined value are selected. Such a homology is at least about 80%, preferably at least 90%, and more preferably at least 95%. The length of a homologous region satisfying the above-mentioned conditions is generally at least 25 mers, and preferably at least 30 mers.

If nucleotide sequences for probes are selected from the above-mentioned point of view, for example in Fig. 1, as common portions between λHC 2256 and λHC 2207, there are mentioned portions from nucleotide numbers 300 to 343, 348 to 412, 414 to 442, 453 to 485, and 498 to 526; as common portions between λHC 2256, λHC 2207 and λHC 2244, there are mentioned portions 678 to 703, 714 to 739, 783 to 847, and 854 to 884. Moreover, in the sequence 277 to 307, the 299th nucleotide is T in λHC 2256, and A in λHC 2207. On the other hand, in the sequence 750 to 781, the 758th nucleotide is C in λHC 2256 and T in both λHC 2207 and λHC 2244. In this case, when probes in which the 299th nucleotide is T in the former case, or the 758th nucleotide is T in the latter case are prepared and used, and if the probes are 30 mers, then the ratio of mismatch is about 3.3% and λHC 2256 can be easily detected. Similarly, if the same thought is applied to 9 clones shown in Fig. 2, there are no sequences of nucleotide sequences of these 9 clones, which sequences are identical in an at least a 30 mer length. Nevertheless, the number of mutations is relatively small in the 30 mer sequence from 1 to 30, the 29 mer sequence from 22 to 50, the 47 mer sequence from 67 to 113, the 37 mer sequence from 155 to 191, the 41 mer sequence from 226 to 266, the 37 mer sequence from 296 to 332, the 32 mer sequence from 340 to 371, the 83 mer sequence from 361 to 443. In addition, for example, since the sequence from 235 to 269 is considered as that having a relatively small number of mutations, it is easily understood that the above-listed sequences are only examples. Among these sequences, for example, when considering the sequences from 22 to 50, the 27th position is A in only λHC 2246, and is G in other clones; the 31th position is T in λCH 2248, λHC 2220 and λHC 2216, and is C in other clones; the 33th position is A in only λHC 2211, and is G in other clones. Since the difference among the clones is only one base, and the ratio of mismatch is about 3.4%, then even though there is no consensus sequence which is a completely match among the clones, the sequences function as probes. Moreover, in sequences from 67 to 103, if the sequence in λHC 2206 is selected as a probe, since the mismatch of nucleotide is only one between the clones, then effective probes having at least a 30 mer length can be constructed.

Where there are some mutations at the same position among the clones, by introducing 5-fluorouracil-(5-FU) or inosine (I) nucleotide to the corresponding position, the function of probe is maintained without affecting the hybridization (Y. Takahashi et al., Proc. Natl. Acad. Sci. USA Vol. 82, 1931-1935 (1985); T. Kodama et al., Nature Vol. 343, 531 (1990)). By using such a technique, the scope of portion which can be used as probes can be further expanded. For example, in regions where two or more nucleotide sequences are in parallel in Figs. 1 and 2, if there is a difference in nucleotides among these nucleotide sequences, by selecting a region having at least a 25 mer length, so that replacement of one of the different nucleotides at the same position with 5-fluorouracil or inosine provides an at least 80% homology between any arbitrarily selected two nucleotide sequences, DNAs which belong to the above-mentioned region and have a nucleotide sequence including said 5-fluorouracil or inosine replacement can be used. In this case, a region which satisfies the above-mentioned condition by a replacement of not more than 4 nucleotides is preferably used. Taking the region from 301 to 330 as an example, 30 mer probes can be constructed by introducing 5-FU or I into the positions 306, 309, 318 and 324.

Moreover, although a portion of each clone is necessarily the best probe for detecting the corresponding clone, if the probe is long enough it can detect a target nucleotide sequence having one or two nucleotide differences. Naturally, a precondition is that the nucleotide sequence of the probe thus selected is specific to non-A, non-B hepatitis; this is the same for the selection of primers. An extent of nucleotide mutations in a nucleotide used for a probe or primer is preferably within 10 to 15% (i.e. at least an 85 to 75% homology) in a nucleotide sequence of the probe or primer, since it is for a specific diagnosis.

In an actual use of these probes for the diagnosis of non-A, non-B hepatitis, it is necessary to detect the probe which has hybridized with nucleic acid in a sample, and accordingly, the prove should be marked with a certain label. As described above, in addition to radioactive label with 32p, well known are non-radioactive labels (Toyozo Takahashi, DNA-prove, Technique and Application, CMC, 1988), and a device for a non-radioactive labeling without damaging the hybridization efficiency is provided for the probes. For example, probes having a high detection efficiency can be constructed by inserting a special linker capable of binding a chemical luminescent substance or fluorescent pigment between nucleotides of nucleotide sequence of a DNA probe (Arnold et al., Clinical Chemistry, Vol. 35, 1588 (1989)).

For a sample containing only a very small amount of a target RNA or DNA, it is difficult to directly detect the RNA or DNA. In such a case, as described above, a region encompassing the target nucleotide sequence can be gene-amplified for a detection thereof. A region to be gene-amplified is usually that having at least a 100 nucleotide length, which is enzymatically gene-amplified using primers.

Although the above-mentioned guideline is provided as one of the standards for the construction of a probe or primers, according to the present invention, it is not necessary to follow this concept, and any probe or primers not conforming to this concept can be used for the detection of a non-A, non-B hepatitis-specific gene. Among the clones shown in Figs. 1 and 2, the preferable probes are shown in Table 2. In addition, as examples of preferable primers, nucleotide sequences shown in Table 3, and complementary nucleotide sequences, can be mentioned.

Note, in the nucleotide sequences of probes shown in Table 2, the position marked "X" is an example of sites for introducing thereinto a linker which will bind a coloring substance, fluorescent substance or chemical luminescent substance when carrying out a detection therewith. Other sites can be used for their insertion.

A linker contains e.g. an $NH_2$ group or a COOH group, to which e.g. a coloring substance, fluorescent substance or a chemical luminescent substance is attached to construct a non-radioactively labeled probe. Linkers are commercially available and, for example, "Amino Modifier II" (Clontech) and "Aminolink II" (Applied Biosystems) may be mentioned. For a 32p-radiolabeled probe, a linker shown by X is not necessary.

Table 2

| Identification | Position in Figs. 1 and 2 | | Length | SEQ ID NO. |
|---|---|---|---|---|
| CP1 | A#2256 | 84-116 | 33 | 19 |
| CP2 | A#2256 | 141-172 | 32 | 20 |
| CP3 | A#2256 | 189-221 | 33 | 21 |
| CP4 | A#2256 | 258-289 | 32 | 22 |
| CP5 | A#2207 | 277-307 | 31 | 23 |
| CP6 | A#2256 | 315-347 | 29 | 24 |
| CP7 | A#2256 | 768-798 | 31 | 25 |
| CP8 | A#2256 | 806-838 | 33 | 26 |
| CP9 | B#2246 | 1-32 | 32 | 27 |
| CP10 | B#2206 | 239-269 | 31 | 28 |
| CP11 | B#2230 | 340-369 | 30 | 29 |
| CP12 | B#2248 | 377-407 | 31 | 30 |

Table 3

| Identification | Position in Figs. 1 and 2 | SEQ ID NO. |
|---|---|---|
| P-70 | A 70-91 | 31 |
| P-139 | A 139-160 | 32 |
| P-277 | A 277-295 | 33 |
| P-315R | A 315-336 | 34 |
| P-317 | A 317-337 | 35 |
| P-447 | A 447-468 | 36 |
| P-447R | A 447-468 | 37 |
| P-489R | A 489-508 | 38 |
| P-549R | A 549-568 | 39 |
| P-705R | A 705-726 | 40 |
| P-708 | A 708-729 | 41 |
| P-774 | A 774-796 | 42 |
| P-774R | A 774-796 | 43 |
| P-942R | B 64-84 | 44 |
| P-1014R | B 136-158 | 45 |
| P-1023 | B 145-167 | 46 |
| P-1041 | B 163-182 | 47 |
| P-1176 | B 298-317 | 48 |
| P-1301R | B 428-447 | 49 |
| P-1335R | B 457-476 | 50 |

The primers marked with "R" are those for synthesis oriented from downstream to upstream, and the primers not marked with "R" are those for synthesis oriented from upstream to downstream.

Methods of extracting nucleic acids from a blood sample and a tissue sample, to identify nucleotides specific to non-A, non-B hepatitis, are well known and are described, for example, in Molecular Cloning: Cold Spring Harbor Laboratory Press (1989), and therefore, there is no difficulty in working with the present invention.

Next, the present invention is explained by Examples, without any limitation of the present invention thereby.

Example 1. Extraction of RNA from serum and liver tissue

RNA was extracted from serum and liver tissue using guanidine thiocyanate (GTC) according to a method of P. Chomczymski et al. (Analytical-Biochemistry, Vol. 162, 156, 1987).

To 1 ml of serum were added 160 µl of 50% polyethylene glycol 4000 and 40 µl of 5M NaCl, and after mixing and allowing the mixture to stand at 4°C for an hour, a precipitate was obtained by centrifugation. Then 100 mg of this precipitate or liver tissue was dissolved in 300 µl of Solution D (4M GTC, 25 mM sodium citrate (pH 7.0), 0.5% Sarcosil, and 100 mM 2-macaptoethanol), and 45 µl of 2M sodium acetate (pH 4), 300 µl of water-saturated phenol and 60 µl of chloroform/isoamylalcohol (49:1) were added thereto to extract RNA. After centrifugation, to an aqueous layer were added 1 µl of 20 µg/µl glycogen and 2.5 volumes of ethanol, and after allowing to stand at -20°C, the mixture was centrifuged to obtain a precipitate. This precipitate was washed with 80% ethanol to obtain RNA. Moreover, RNA was obtained by adding 1 ml of GTC solution to 100 µl of serum.

Example 2 Amplification and detection of a non-A, non-B hepatitis-specific gene corresponding to a cDNA inserted into recombinant phage λHC 2232, λHC 2230, λHC 2206, λHC 2258, λHC 2248, λHC 2220, λHC 2246, λHC 2211 or λHC 2216

Among recombinant phages containing a cDNA coding for non-A, non-B hepatitis-specific antigen protein, λHC 2232, λHC 2230, λHC 2206, λHC 2258, λHC2248, λHC 2220, λHC 2246, λHC 2211 and λHC 2216 are recognized to have a high homology. To detect genes corresponding to these cDNAs oligonucleotides having the following nucleotide sequences were synthesized as primes and probes from a region having an especially high homology among nucleotide sequences of the cDNAs. The synthesis was carried out using a DNA automatic synthesizer (Applied Biosystems; 380A Type).

Primer (PR)

PR1: 5'-CTGCACAGGTACGCTCCGGC-3' (SEQ ID NO: 51) (Nucleotide sequence 31 - 50 of the cDNA inserted into λHC 2232)
PR2: 5'-CTCCTGCGGGATGAGGTCAC-3' (SEQ ID NO: 52) (Nucleotide sequence 61 - 80 of the cDNA inserted into λHC 2232)
PR3: 5'-GGCGCAGACAACTGACTAGC-3' (SEQ ID NO: 53) (Nucleotide sequence 250 - 269 of the cDNA inserted into λHC 2232)
PR4: 5'-CCGCCCATCTCCTGCCGCCA-3' (SEQ ID NO: 54) (Nucleotide sequence 340 - 359 of the cDNA inserted into λHC 2232)

Probe (PB)

PB1: 5'-CACAGCTCCCATGTGAGCCCGAACCGGATG-3' (SEQ ID NO: 55) (Nucleic acid sequence 116 - 145 of the cDNA inserted into λHC 2258)

The RNA prepared in Example 1 and 0.5 µg of a random primer were mixed, and after carrying out an annealing at 65°C for 5 minutes according to a convertional procedure (J. Sambrook et al., Molecular Cloning; Cold Spring Habor Laboratory Press (1989)), a reaction was carried out at 37°C for one hour using 100 units of reverse transcriptase (Takara Shuzo), to synthesize a single-stranded complementary DNA (scDNA). To the resulting cDNA were added 100 p mol each of primers PR1 and PR4, and a gene amplification was carried out by the PCR method using a DNA Amplification System (Takara Shuzo) and Taq DNA polymerase. The reaction comprised heating at 92°C for 3 minutes, followed by a 30 to 40 times repetition of a reaction comprising heating at 92°C for 2 minutes, at 40°C for 2 minutes, and at 72°C for 2 minutes.

Then 10 µl of the above PCR reaction mixture was heated to 100°C and rapidly cooled for 5 minutes, and after a hybridization buffer (60 mM Tris-HCl (pH 8.0), 160 mM NaCl, 6.2 mM MgCl) and a 5'-32p-labeled probe were added thereto, heated at 60°C for 15 minutes. The mixture was subjected to column chromatography using Sephadex G-75 (Pharmacia) equilibrated with 10 mM Tris-HCl (pH 7.5), 300 mM NaCl and 1 mM EDTA, and a DNA fraction of about 330 bases corresponding to a non-A, non-B hepatitis-specific gene which is expected to be gene-amplified by the PCR method, was obtained, and the radioactivity thereof was then measured. As a result, a radioactivity was not detected for RNA derived in a serum of a healthy person, but when similarly treated, was detected in an RNA extracted from serum and liver tissue of a non-A, non-B hepatitis patient, confirming the presence of a non-A, non-B hepatitis-specific gene specifically amplified.

Moreover, primers PR2 and PR3 were added to the above PCR reaction mixture, and 30 to 40 cycles of gene-amplification were similarly carried out. The resulting reaction mixture was subjected to electrophoresis using a 8% polyacrylamide gel, and thereafter, the gel was stained. As a result, when RNA extracted from serum and liver tissue of a non-A, non-B hepatitis patient was used, a band of about 200 bp specifically gene-amplified by primers PR2 and PR3 was found, but was not detected in RNA derived from the serum of a healthy person and similarly treated, confirming the presence of a non-A, non-B hepatitis-specific gene specifically amplified, from the non-A, non-B hepatitis patient.

Example 3. Amplification and detection of a non-A, non-B hepatitis-specific gene corresponding to a cDNA inserted into the recombinant phages λHC 2256, λHC 2244 and λHC 2207

Among recombinant phages containing a cDNA coding for non-A, non-B hepatitis-specific antigen protein, to detect a gene corresponding to cDNAs of λHC 2256, λHC 2244 and λHC 2207, similar to Example 2, oligonucleotides having the following nucleotide sequences were synthesized as primers and probes.

Primer (PR)

PR5: 5'-ACATCCTGGCGGGTTATGGAGC-3' (SEQ ID NO: 56) (Nucleotide sequence 70 - 91 of the cDNA inserted into λHC 2256)
PR6: 5'-AGTCCCAAACATCCCTGAGCCA-3' (SEQ ID NO: 57) (Nucleotide sequence 447 - 468 of the cDNA inserted into λHC 2256)
PR7: 5'-TGCCCTCCACCGAGGACCTGG-3' (SEQ ID NO: 58) (Nucleotide sequence 139 - 160 of the cDNA inserted into λHC 2256)

PR8: 5'-CGCTTTCAGGCACATAATGCGT-3' (SEQ ID NO: 59) (Nucleotide sequence 315 - 336 of the cDNA inserted into λHC 2256)

Probe (PB)

PB2: 5'-TGATAGCGTTCGCTTCGCGGGGTAACCACGT-3' (SEC ID NO: 60) (Nucleotide sequence 277 - 307 of the cDNA inserted into λHC 2256)

The RNA prepared in Example 1 and 0.5 µg of a random primer were mixed, and scDNA was synthesized as in Example 2. To the resulting scDNA were added primers PR5 and PR6, and after carrying out a gene-amplification as in Example 2, detection of an amplified non-A, non-B hepatitis-specific gene was attempted by hybridization with probe PB2. As a result, where RNA extracted from a non-A, non-B hepatitis patient was used, specific radioactivity was detected, confirming the presence of a specifically amplified non-A, non-B hepatitis-specific gene.

Moreover, to the above-mentioned PCR reaction mixture were added primers PR7 and PR8, and 30 to 40 cycles of a gene amplification were similarly carried out. The resulting reaction mixture was subjected to a 8% polyacrylamide gel electrophoresis, and thereafter, the gel was stained with ethidium bromide. As a result, where RNA extracted from the serum or liver tissue of a non-A, non-B hepatitis patient was used, a band of about 150 bp specifically gene-amplified using primers PR7 and PR8 was detected, confirming the presence of a specifically amplified non-A, non-B hepatitis specific gene. Note, where a similarly treated RNA derived from a serum of a healthy person was used, the 150 bp band was not detected.

Example 4. Detection of a specifically amplified non-A, non-B hepatitis-specific gene by a probe incorporating a chemical luminescent substance

A one-tenth volume of the reaction mixture obtained by gene-amplification in Example 3 was heated at 95°C for 5 minutes and then rapidly cooled, and to the mixture was added the probe CP5 shown in Table 2 (X in the nucleotide sequence of the probe represents the position of a linker (Aminomodifier II/Clontech) and this linker had been labeled with acridinum ester) diluted with a hybridization solution (0.1 M NaCl, 10 mM Tris-HCl(pH 6.0), 1 mM EDTA), followed by incubation at 60°C for 30 minutes. To this was added 200 µl of 0.1 M sodium borate (pH 7.6), and after an incubation at 60°C for 10 minutes and hydrolysis of acridinium ester in a probe not hybridizing with the target, $H_2O_2$ and NaOH solutions were added to the mixture and an intensity of the chemical luminescence was measured by a lumino meter (see, Arnold et al., supra). As a result, where RNA extracted from the serum and liver tissue of a non-A, non-B hepatitis patient was used, a specific chemical luminescene was measured and a non-A, non-B hepatitis-specific gene was quickly detected, while for a similarly treated RNA derived from a healthy person, a chemical luminescence indicating the presence of non-A, non-B hepatitis-specific gene was not detected.

As seen from the above, by using primers and probe oligonucleotides prepared on the basis of the nucleotide sequences of a cDNA coding for a non-A, non-B hepatitis-specific protein, non-A, non-B hepatitis-specific genes contained in RNA extracted from a non-A, non-B hepatitis patient can be detected.

Reference to microorganisms deposited under Rule 13 - 2 and depository authority:

The Ministry of International Trade and Industry, Fermentation Research Institute, Agency of Industrial Science and Technology

Address: 1-3 Higashi 1-chome Tsukuba-shi Ibaraki Japan

Deposition No. and deposition date:

| | | |
|---|---|---|
| 1. | FERM P-10842 | July 13, 1989 |
| 2. | FERM P-10843 | July 13, 1989 |
| 3. | FERM P-10844 | July 13, 1989 |
| 4. | FERM P-10847 | July 13, 1989 |
| 5. | FERM P-10852 | July 18, 1989 |
| 6. | FERM P-10854 | July 18, 1989 |
| 7. | FERM P-10855 | July 18, 1989 |
| 8. | FERM P-10856 | July 18, 1989 |
| 9. | FERM P-10857 | July 18, 1989 |
| 10. | FERM P-10859 | July 18, 1989 |
| 11. | FERM P-10860 | July 18, 1989 |

| | | |
|---|---|---|
| 12. | FERM P-10861 | July 18, 1989 |
| 13. | FERM P-10878 | July 21, 1989 |
| 14. | FERM P-10881 | July 21, 1989 |
| 15. | FERM P-10882 | July 21, 1989 |
| 16. | FERM P-10883 | July 21, 1989 |
| 17. | FERM P-10889 | July 26, 1989 |
| 18. | FERM P-10892 | July 26, 1989 |
| 19. | FERM P-10893 | July 26, 1989 |
| 20. | FERM P-10894 | July 26, 1989 |
| 21. | FERM P-10896 | July 26, 1989 |
| 22. | FERM P-10897 | July 26, 1989 |
| 23. | FERM P-10898 | July 26, 1989 |
| 24. | FERM P-10899 | July 26, 1989 |
| 25. | FERM P-10900 | July 26, 1989 |
| 26. | FERM P-10904 | July 28, 1989 |
| 27. | FERM P-10905 | July 28, 1989 |
| 28. | FERM P-10906 | July 28, 1989 |
| 29. | FERM P-10908 | July 28, 1989 |
| 30. | FERM P-10911 | July 28, 1989 |
| 31. | FERM P-10917 | August 2, 1989 |
| 32. | FERM P-10919 | August 2, 1989 |
| 33. | FERM P-10920 | August 2, 1989 |
| 34. | FERM P-10921 | August 2, 1989 |
| 35. | FERM P-10922 | August 2, 1989 |
| 36. | FERM P-10923 | August 2, 1989 |
| 37. | FERM P-10931 | August 9, 1989 |
| 38. | FERM P-10932 | August 9, 1989 |
| 39. | FERM P-10933 | August 9, 1989 |
| 40. | FERM P-10934 | August 9, 1989 |
| 41. | FERM P-10935 | August 9, 1989 |
| 42. | FERM P-10936 | August 9, 1989 |
| 43. | FERM P-10937 | August 9, 1989 |
| 44. | FERM BP-2951 | July 13, 1989 |
| 45. | FERM BP-2952 | July 13, 1989 |
| 46. | FERM BP-2953 | July 13, 1989 |
| 47. | FERM BP-2954 | July 18, 1989 |
| 48. | FERM BP-2955 | July 18, 1989 |

| | | |
|---|---|---|
| 49. | FERM BP-2956 | July 21, 1989 |
| 50. | FERM BP-2957 | July 21, 1989 |
| 51. | FERM BP-2958 | July 21, 1989 |
| 52. | FERM BP-2959 | July 21, 1989 |
| 53. | FERM BP-2960 | July 26, 1989 |
| 54. | FERM BP-2961 | July 26, 1989 |
| 55. | FERM BP-2962 | July 26, 1989 |
| 56. | FERM BP-2963 | July 28, 1989 |
| 57. | FERM BP-2964 | July 28, 1989 |
| 58. | FERM BP-2965 | July 28, 1989 |
| 59. | FERM BP-2966 | August 2, 1989 |
| 60. | FERM BP-2967 | August 2, 1989 |
| 61. | FERM BP-2968 | August 9, 1989 |

SEQUENCE LISTING

SEQ ID NO: 1

Length: 668

Sequence Type: Nucleic acid

Strandness: Double strand

Topology: Linear

Molecule Type: cDNA

Source

Organism: Human

Immediate Source: Phage clone λHC 2211

Sequence

```
GAATTCTTCA CGGAATTGGA TGGGGTGCGG CTACACAGGT ACGCTCCGGC  50
GTGCAAGCCT CTCCTACGGG ATGAGGTCAC ATTCCAGGTC GGGCTCAACC 100
AATTCCCGGT TGGGTCACAG CTCCCATGTG AACCCGAACC GGATGTAATG 150
GTGGTCACCT CTATGCTCAC CGACCCCTCC CATATTACAG CAGAGACGGC 200
TAAGCGTAGG CTGGCCAGAG GGTCTCCCCC TTCTTTGGCC AGCTCTTCAG 250
CTAGTCAGTT GTCTGCGCCC TCCTTGAAGG CGACATGCAC CACCCGTCAT 300
GACTCCCCGG ACGCTGACCT CATAGAGGCC AACCTCCTGT GGCGGCAGGA 350
GATGGGCGGG AACATCACCC GTGTGGAGTC AGAGAATAAG GTAGTGATTT 400
TGGACTCTTT TGAACCGCTT CGGGTGGAGG AGGATGAGAG GGAAGTATCC 450
GTAGCGGCGG ATTTCAGTGA CTTGAATGCA GAATGAATCC CGTGGCTCAC 500
TTCCTAGACT ATTTGCCAAA GAAGATGTTG CCCTGGCCAT GATCAAGATG 550
ACACAAACGG TGGCCTTTTG CAGGGAGAAC CGCCGTGGAG GCCTGTGTCT 600
GTGGCACTGG TAGCTTCTCT CTGCAGGCAA AGACCCCATG GCTTAGTTCT 650
TCATCAGAGT GAGAATTC                                    668
```

SEQ ID NO: 2

Length: 479

Sequence Type: Nucleic acid

Strandness: Double strand

Topology: Linear

Molecule Type: cDNA

Source

Organism: Human

Immediate Source: Phage clone λHC 2246

Sequence

```
GAATTCTTCA CGGAGTTGGA TGGGGTACGG CTGCACAGGT ACGCTCCGGC  50
GTGCAAGCCA CTCCTACGGG ATGAGGTCAC ATTCCAGGTC GGGCTCAACC 100
AATTTCCAGT TGGATCACAG CTCCCATGTG AGCCCGAGCC GGATGTAGCG 150
GTGCTCACTT CCATGCTCAC CGACCCCTCC CACATTACAG CAGAGACGGC 200
TAAGCGTAGG CTGGCCAGGG GGTCCCCCCC CTCCTTGGCC AGCTCTTCAG 250
CTAGTCAGTT GTCTGCGCCC TCCTTGAAGG CGACATGCAC TACCCACCAT 300
GACTCCCCGG ACGCTGACCT CATCGAGGCC AACCTCCTGT GGCGGCAGGA 350
GATGGGAGGA AACATCACCC GCGTGGAGTC AGAGAATAAG GTAGTAATTC 400
TAGACTCTTT TGACCCGCTC CGAGCGGAGG AGGATGAGAG GGAAGTGTCC 450
GTTGCGGCGG AGATCCTGCG GAAGACCAG                        479
```

SEQ ID NO: 3

Length: 498

Sequence Type: Nucleic acid

Strandness: Double strand

Topology: Linear

Molecule Type: cDNA

Source

Organism: Human

Immediate Source: Phage clone λHC 512

Sequence

TCACTCAATC CTCGACGGTG CTGCCGGTGC GGCAATCCGG AACGATACCG 50
ACGCCGGATC GCCCTGCTGC CCCCACGCAT TTACCGCCCG GACTGTCAGC 100
CTGTAGTTCC CCAGCGCCAG TTGCGTGAAG CGGTATGTGG TTTCCGTCGT 150
CCGGGCCGTG CTGACCAGCC GCTCACTGCC GTCGTCCGTG TTACGGTCAG 200
ACGGAGCAGG AAACTCACGC CTTCACACTT CGGTGTGTCC CATCGCGCCA 250
GCACCTGATA TTCCCCGCTG TCTGCAGTGA CTTCTGCGGT CAGGTGCTGC 300
ACCGCTCGTG ACACCATTCA CCGTGCCACT CTGTTCGCCG TCAAAGTGCG 350
CCCCGTTATC CACGATGGCC TCTTTTTCCG GCACATGCTG CACGGCGGTG 400
ATGGCATACG TGCCGTCGTC GTTCTCACGG ATACTCACGC AGCGGAACAG 450
TCCTGGCGCA GCGTCGGCAG CTTCAGCTCC CATACGCTGT ATTCAGCT 498

SEQ ID NO: 4

Length: 685

Sequence Type: Nucleic acid

Strandness: Double strand

Topology: Linear

Molecule Type: cDNA

Source

Organism: Human

Immediate Source: Phage clone λHC 2206

Sequence

GAATTCTTCA CAGAGTTGGA CGGGGTGCGG CTGCACAGGT ACGCTCCGGC 50
GTGCAGACCT CTCCTGCGGG ATGAGGTCAC ATTCCAGGTC GGGCTCAACC 100
AATACCCGGT TGGGTCACCG CTCCCATGTG AGCCCGAACC GGATGTAACA 150
GTGGTCACTT CCATGCTCAC CGACCCCTCC CACATTACAG CGGAAACTGC 200
CAGGCGTAGG TTGGCCAGGG GGAGTCCCCC TTCCTTGGCC AGCTCTTCAG 250
CTAGTCAGTT GTCTGCACCT TCCTTGAAGG CGACATGTAC TACCCATCAT 300
GACTCTCCAG ACGCTGATCT CATCGAGGCC AACCTTCTAT GGCGGCAGGA 350
GATGGGCGGG AACATCACCC GTGTGGAGTC AGAGAATAAG GTAGTAATCC 400
TAGACTCTTT TGACCCGCTT CGAGCGGAGG AGGATGAGAG GGAAGTATCC 450
GTTGCGGCGG AGATCCTGCG GAGAACCAGG AGATTCCCCC CGGCGATACC 500
TGTATGGGCG CGCCCGGACT ACAACCCGCC ACTGATAGAA TCTTGGAAGG 550
ACCCAGACTA CGTCCCACCG GTGGTACACG GGTGTCCATT GCCACCTGCC 600
AAGACCCCTC AAGTGGATAT TCAGACCTCT TTGAGGCTTT CGTTGGAAAC 650
GGGATTTCTT CATACTATGC TAGACAGAAG AATTC 685

SEQ ID NO: 5

Length: 608

Sequence Type: Nucleic acid

Strandness: Double strand

Topology: Linear

Molecule Type: cDNA

Source

Organism: Human

Immediate Source: Phage clone λHC 2207

Sequence

TGATAGCGTT CGCTTCGCGG GGAAACCACG TCTCCCCCAC GCACTATGTG 50

CCTGAAAGCG ACGCTGCAGC GCGTGTCACC CAGATCCTCT CCAGCCTTAC 100
CATCACTCAG CTGTTGAAGA GGCTCCACCA GTGGATCAAT GAGGACTGCT 150
CCACGCCATG CTCCGGTTCG TGGCTTAGGG ATGTTTGGGA CTGGATATGC 200
ACGGTGTTGA CTGACTTCAA AACCTGGCTC CAGTCCAAGC TCCTGCCGCG 250
ATTGCCGGGA GTCCCTTTCC TTTCATGCCA ACGAGGGTAC AAGGGAGTCT 300
GGCGGGGAGA TGGTGTCATG CAAACCACCT GCCCATGTGG AGCACAGATC 350
AGTGGGCATG TCAAAAATGG CTCCATGAGG ATCGTTGGGC CTAGAACCTG 400
TAGCAACACG TGGCACGGAA CGTTCCCTAT CAACGCGTAC ACCACAGGCC 450
CCTGCACACC CTCCCCGGCG CCCAACTACT CTAGGGCGTT GTGGCGGGTG 500
GCTGCTGAGG AGTACGTGGA GGTCACGCGG GTGGGGGATT TCCACTACGT 550
GACGGGCATG ACCACTGACA ACGTAAGATG CCCATGCCAG GTTCCGGCCC 600
CCGAATTC 608

SEQ ID NO: 6

Length: 473

Sequence Type: Nucleic acid

Strandness: Double strand

Topology: Linear

Molecule Type: cDNA

Source

Organism: Human

Immediate Source: Phage clone λHC 2216

Sequence

GAATTCTTCA CAGAGTTGGA TGGGGTGCGG TTGCACAGGT ACGCTCCGGC 50
TGCAAAGCCT CTCCTACGGG ATGAGGTCAC ATTTCAGGTC GGGCTCAACC 100
AATTCCCGGT TGGGTCACAG CTCCCATGTG AGCCCGAACC GGATGTAACA 150

GTGATCACCT CCATGCTCAC CGACCCCTCC CACATTACAG CAGAGGCGGC 200

TGGGCGTAGG CTGGCCAGAG GGTCTCCCCC TTCCTTGGCC AGCTCTTCGG 250

CTAGTCAGTT GTCTGCGCCC TTCCCTGTTG AAGGCCGACA TGCACTACCC 300

GTCATGACTC CCCAGACGCT GACCTCATCG AGGCCAATCT CCTGTGGCGG 350

CAGGAGATGG GAGGGAACAT CACCCGCGTG GAGTCAGAGA ACAAGGTACT 400

AATCCTAGAC TCTTTTGACC CGCTCCGAGC GGAGGAGGAT GAGAGGGAGA 450

TATCTGTTGC GGCCCAGCTG AGC 473

SEQ ID NO: 7

Length: 526

Sequence Type: Nucleic acid

Strandness: Double strand

Topology: Linear

Molecule Type: cDNA

Source

Organism: Human

Immediate Source: Phage clone λHC 2220

Sequence

GAATTCTTCA CAGAGCTGGA TGGGGTGCGG TTGCACAGGT ACGCTCCGGC 50

GTGCAAGCCT CTCCTACGGG ATGAGGTCAC ATTTCAGGTC GGGCTCAACC 100

AATTCCCGGT TGGGTCACAG CTCCCGTGTG AGCCCGAACC GGATGTAACG 150

GTGATCACTT CCATGCTCAC CGACCCCTCC CACATTACAG CAGAGACGGC 200

TGGGCGTAGG CTGGCCAGAG GGTCTCCCCC TTCCTTGGCC AGCTCTTCGG 250

CTAGTCAGTT GTCTGCGCCC TCCTTGAAGG CAACATGCAC TACCCGTCAT 300

GACTCCCCAG ACGCTGACCT CATCGAGGCC AATCTCCTGT GGCGGCAGGA 350

GATGGGAGGG AACATCACCC GCGTGGAGTC AGAGAACAAG GTAGTAATCC 400

TAGACTCTTT TGACCCGCTC CGAGCGGAGG AGGATGAGAG GGAGATATCT 450
GTTGCGGCGG AGATCCTACG GAAATCTAGG AAATTCCCCC CAGCATTACC 500
CATATGGGCG CGCCCGGACT ACAACC 526

SEQ ID NO: 8

Length: 599

Sequence Type: Nucleic acid

Strandness: Double strand

Topology: Linear

Molecule Type: cDNA

Source

Organism: Human

Immediate Source: Phage clone λHC 2230

Sequence

GAATTCTTCA CAGAGTTGGA TGGGGTGCGG CTGCACAGGT ACGCTCCGGC 50
GTGCAAACCT CTCCTGCGGG ATGAGGTCAC GTTCCAGGTC GGGCTCAACC 100
AATACCCGGT TGGATCACAG CTCCCATGCG AGCCCGAACC GGATGTGGCG 150
GTGCTCACTT CCATGCTCAC CGACCCCACC CACATTACAG CAGAAGCGGC 200
TAGGCGCAGG CTGGCCAGAG GGTCTCCTCC TTCCTTGGCC AGCTCTTCAG 250
CTAGTCAGTT GTCTGCGCCC TCCTTGAAGG CGACATGCAC TACCCATCAT 300
GACTCCCCAG ACGCTGACCT CATCGAGGCC AACCTCCTGT GGCGGCAGGA 350
GATGGGCGGA AACATCACCC GCGTGGAGTC AGAGAATAAG GTAGTAATTC 400
TAGACTCTTT TGAACCGCTT CGAGCGGAAG AGGATGAGAG GGAAGTATCC 450
GTTGCGGCAG AGATCCTGCG GAAAACCAGG AGATTCCCCG CAGCGATGCC 500
CATATGGGCA CGTCCGGACT ACAACCCACC ATTACTACAG TCCTGGAAGG 550
ACCCGGACTA CGTCCCTCCG GTGGTGCACG GGTGCCCATT GCCACCTGC 599

SEQ ID NO: 9

Length: 1184

Sequence Type: Nucleic acid

Strandness: Double strand

Topology: Linear

Molecule Type: cDNA

Source

Organism: Human

Immediate Source: Phage clone λHC 2232

Sequence

GAATTCTTCA CAGAGTTGGA TGGGGTGCGG CTGCACAGGT ACGCTCCGGC 50
GTGCAAACCT CTCCTGCGGG ATGAGGTCAC GTTCCAGGTC GGGCTCAACC 100
AATACCCGGT TGGATCACAG CTCCCATGCG AGCCCGAACC GGATGTGGCG 150
GTGCTCACTT CCATGCTCAC CGACCCCACC CACATTACAG CAGAAGCGGC 200
TAGGCGCAGG CTGGCCAGAG GGTCTCCTCC TTCCTTGGCC AGCTCTTCAG 250
CTAGTCAGTT GTCTGCGCCC TCCTTGAAGG CGACATGCAC TACCCATCAT 300
GACTCCCCAG ACGCTGACCT CATCGAGGCC AACCTCCTGT GGCGGCAGGA 350
GATGGGCGGA AACATCACCC GCGTGGAGTC AGAGAATAAG GTAGTAATTC 400
TAGACTCTTT TGAACCGCTT CGAGCGGAAG AGGATGAGAG GGAAGTATCC 450
GTTGCGGCAG AGATCCTGCG GAAAACCAGG AGATTCCCCG CAGCGATGCC 500
CATATGGGCA CGTCCGGACT ACAACCCACC ATTACTACAG TCCTGGAAGG 550
ACCCGGACTA CGTCCCTCCG GTGGTGCACG GGTGCCCATT GCCACCTGCC 600
AAGGCCCCTC CAGTACCACC TCCAAGGAGA AAGAGGACGG TTGTCCTGAC 650
AGAATCCACC GTGTCTTCCG CCTTGGCGGA GCTTGCTACA AAGACCTTCG 700
GCGGGTCCGG ATCATCGGCC GCCGACAGCG GCACAGCAAG CGGCCCTCCT 750
GGCCAGGCCT CCGACGATGG AGATACAGGA TCCGACGTTG AGTCGTACTC 800

CTCCATGCCC CCCCTTGAGG GAGAGCCGGG GGACCCCGAC CTCAGCGACG 850
GGTCTTGGTC TACTGTGAGT GAGGAGGCTG GTGAGGATGT CGTCTGCTGC 900
TCGATGTCCT ACACATGGAC AGGCGCCTTA ATCACACCAT GCACCGCGGA 950
GGAGAGCAAG CTGCCCATCA ACCCGTTGAG CAACTCTTTG CTGCGGGCAT 1000
CTGCTCGGGC GTATCATCAA CTGATGAGCA AGAAGGATAT AATTCCTACG 1050
CCCTCTCAGC CGATGAACAG TTGGAATAGG TTGTTAGCGG TAACTAAGAT 1100
TAGTATGGTA ATTAGGAAAA TGAGTAGATA TTTGAAGAAC TGATTAATGT 1150
TTGGGTCTGA GTTTATATAT CACAGTGAGA ATTC 1184

SEQ ID NO: 10

Length: 255

Sequence Type: Nucleic acid

Strandness: Double strand

Topology: Linear

Molecule Type: cDNA

Source

Organism: Human

Immediate Source: Phage clone λHC 2244

Sequence

GGGCATGTCA AAAATGGCTC CATGAGGATC GTTGGGCCTA GAACCTGTAG 50
CAACACGTGG CACGGAACGT TCCCTATCAA CGCGTACACC ACAGGCCCCT 100
GCACACCCTC CCCGGCGCCC AACTACTCTA GGGCGTTGTG GCGGGTGGCT 150
GCTGAGGAGT ACGTGGAGGT CACGCGGGTG GGGGATTTCC ACTACGTGAC 200
GGGCATGACC ACTGACAACG TAAGATGCCC ATGCCAGGTT CCGGCCCCCG 250
AATTC 255

SEQ ID NO: 11

Length: 553

Sequence Type: Nucleic acid

Strandness: Double strand

Topology: Linear

Molecule Type: cDNA

Source

Organism: Human

Immediate Source: Phage clone λHC 2248

Sequence

GAATTCTTCA CAGAGTTGGA TGGGGTGCGG TTGCACAGGT ACGCTCCGGC 50
GTGCAAACCT CTCCTACGGG ATGAGGTCAC ATTTCAGGTC GGGCTCAACC 100
AATTCCCGGT TGGGTCACAG CTCCCATGTG AGCCCGAACC GGATGTAACA 150
GTGATCACCT CCATGCTCAC CGACCCCTCC CACATTACAG CAGAGACGGC 200
TGGGCGTAGG CTGGCCAGAG GGTCTCCCCC TTCCTTGGCC AGCTCTTCGG 250
CTAGTCAGTT GTCTGCGCCT TCCTTGAAGG CGACATGCAC TACCCGTCAT 300
GACTCCCCAG ACGCTGACCT CATCGAGGCC AATCTCCTGT GGCGGCAGGA 350
GATGGGAGGG AACATCACCC GCGTGGAGTC AGAGAACAAG GTAGTAATCC 400
TAGACTCTTT TGACCCGCTC CGAGCGGAGG AGGATGAGAG GGAGATATCT 450
GTTGCGGCGG AGATCCTACG GAAATCTAGG AAATTCCCCC CAGCATTACC 500
CATATGGGCG CGCCCGGACT ACAACCCACC ACTGCTAGAG TCTTGGCCAG 550
CTG 553

SEQ ID NO: 12

Length: 884

Sequence Type: Nucleic acid

Strandness: Double strand

Topology: Linear

Molecule Type: cDNA

Source

Organism: Human

Immediate Source: Phage clone λHC 2256

Sequence

```
CGGCTTTCGT GGGCGCCGGC ATAGCCGGCG CGGCTGTTGG CAGCATAGGC  50
CTTGGGAAGG TGCTTGTGGA CATCCTGGCG GGTTATGGAG CAGGGCTGGC 100
AGGCGCACTC GTGGTCTTTA AGGTTATGAG TGGCGACATG CCCTCCACCG 150
AGGACCTGGT CAACTTACTC CCTGCCATCC TTTCCCCTGG CGCCCTGGTC 200
GTCGGGGTCG TGTGCGCACA GATACTGCGT CGACATGTCG GCCCAGGGGA 250
GGGAGCTGTG CAGTGGATGA ACCGGCTGAT AGCGTTCGCT TCGCGGGGTA 300
ACCACGTCTC CCCCACGCAT TATGTGCCTG AAAGCGACGC TGCGAGTCGT 350
GTCACCCAGA TCCTCTCCAG CCTTACCATC ACTCAGCTGT TGAAGAGGCT 400
CCACCAGTGG ATTAATGAGG ACTGCTCCAC GCCATGCTCC GGCACGTGGC 450
TCAGGGATGT TTGGGACTGG ATATGCACGG TGTTGGCTGA CTTCAAGACC 500
TGGCTCCAGT CCAAGCTCCT GCCGCGGTTA CCGGGGGTCC CTTTCCTCTC 550
ATGTCAGCGT GGGTACAAGG GAGTTTGGCG GGGAGATGGC ATCATGCACA 600
CCACCTGCCC ATGCGGAGCC CAAATCACCG GACATGTCAA AAACGGGTCC 650
ATGAGGATCG CCGGGCCTAA AACCTGCAGC AACACGTGGC ACGGAACGTT 700
CCCCATTAAC GCATACACCA CAGGCCCCTG CACACCCTCT CCGGCGCCAA 750
ACTACTCCAG GGCGTTGTGG CGGGTGGCTG CGGAGGAGTA CGTGGAGGTC 800
ACGCGGGTGG GGGATTTCCA CTACGTGACG GGCATGACCA CTGACAATGT 850
AAAATGCCCA TGCCAGGTTC CGGCCCCCGA ATTC                  884
```

SEQ ID NO: 13

Length: 524

Sequence Type: Nucleic acid

Strandness: Double strand

Topology: Linear

Molecule Type: cDNA

Source

Organism: Human

Immediate Source: Phage clone λHC 2258

Sequence

```
GAATTCTTCA CAGAGTTGGA CGGGGTGCGG CTGCACAGGT ACGCTCCGGC  50
GTGCAGACCT CTCCTGCGGG ATGAGGTCAC ATTCCAGGTC GGGCTCAACC 100
AATACCCGGT TGGGTCACAG CTCCCATGTG AGCCCGAACC GGATGTAACA 150
GTGGTCACTT CCATGCTCAC CGACCCCTCC CACATTACAG CGGAAACTGC 200
CAGGCGTAGG TTGGCCAGGG GGAGTCCCCC TTCCTTGGCC AGCTCTTCAG 250
CTAGTCAGTT GTCTGCACCT TCCTTGAAGG CGACATGTAC TACCCATCAT 300
GACTCTCCAG ACGCTGATCT CATCGAGGCC AACCTTCTAT GGCGGCAGGA 350
GATGGGCGGG AACATCACCC GTGTGGAGTC AGAGAATAAG GTAGTAATCC 400
TAGACTCTTT TGACCCGCTT CGAGCGGAGG AGGATGAGAG GGAAGTATCC 450
GTTGCGGCGG AGATCCTGCG GAGAACCAGG AGATTCCCCC CGGCGATACC 500
TGTATGGGCG CGCCCGGACC AGCT                             524
```

SEQ ID NO: 14

Length: 174

Sequence Type: Nucleic acid

Strandness: Double strand

Topology: Linear

Molecule Type: cDNA

Source

Organism: Human

Immediate Source: Phage clone λHC 2270

Sequence

GAATTCAGCA AAGTTTCCAG ATACAAGATT AATGGACACA AATCAGTAGC 50

TCTTCCATAC ATCAACAGCT ACCAAGCAGA GAATCACATC AAGAACTCAA 100

CCCCTTTTAC AATAGCTGCG ACAAACAACA ACAACAAAAA AACAAAACTT 150

AGGAATATAC CTAGCAAAGA ATTC 174

SEQ ID NO: 15

Length: 135

Sequence Type: Nucleic acid

Strandness: Double strand

Topology: Linear

Molecule Type: cDNA

Source

Organism: Human

Immediate Source: Phage clone λHC 2410C

Sequence

GAATTCCCTA GCCTGGGCAA CAGAGTGAGA GTCCGTCTCA AAAAAAAAAA 50

AACAACAACA AAAAAAACAA ACCCACAAAA CTGCAGCCAC CTATGTCCCT 100

ACCTCCCCAG CCTCCAGGGC CCCTTCCGGA ATTCC 135

SEQ ID NO: 16

Length: 306

Sequence Type: Nucleic acid

Strandness: Double strand

Topology: Linear

Molecule Type: cDNA

Source

Organism: Human

Immediate Source: Phage clone λHC 2533

Sequence

GAATTCGCGG GAACCCGGGA GGCGGACGTT GCAGTGAGCC GAGATCGCGC 50

CACTGCACTC CAGCCTGGGC GACAGAGCAA GACTCTGTCT CAAAAAAAAA 100

AAAACAAAAA CAAAAAGAAG GACTGGGAGG GTCGGCAGTA ATCGAGGACC 150

ACCTGGCAGT GACAGAGGGT GACCCAGGGC TGGGAGGATA CCCCAGGGGA 200

GACCCCAGGC TCTGAAAAGT GCCTTGCCAT TCAATCTACT TCAGTAATAG 250

CATGTGTCAT GGGATAGATA ATAAAATCCG GAGGGGAAAA AATGCTCGCG 300

GAATTC 306

SEQ ID NO: 17

Length: 174

Sequence Type: Nucleic acid

Strandness: Double strand

Topology: Linear

Molecule Type: cDNA

Source

Organism: Human

Immediate Source: Phage clone λHC 2607

Sequence

GAATTCAGCA CAGTTTCCAG ATACAAGATT AATGGACACA AATCAGTAGC 50

TCTTCCATAC ATCAACAGCT ACCAAGCAGA GAATCACATC AAGAACTCAA 100

CCCCTTTTAC AATAGCTGCG ACAAACAACA ACAACAAAAA AACAAAACTT 150

AGGAATATAC CTAGCAAAGA ATTC 174

SEQ ID NO: 18

Length: 95

Sequence Type: Nucleic acid

Strandness: Double strand

Topology: Linear

Molecule Type: cDNA

Source

Organism: Human

Immediate Source: Phage clone λHC 2610

Sequence

GAATTCCCTA AAAAAGAAAA AAAAAAAAAA AGACTTCAGC CAACAGATCA 50

GAACGCAGAA AATGCATTTG CCTCAGTAGT GAGTCGGCAG AATTC 95

SEQ ID NO: 19

Length: 33

Sequence Type: Nucleic acid

Strandness: Single strand

Topology: Linear

Molecule Type: Synthetic DNA

Sequence

TATGGAGCAG GGGXTGGCAG GCGCACTCGT GGTC 33

SEQ ID NO: 20

Length: 32

Sequence Type: Nucleic acid

Strandness: Single strand

Topology: Linear

Molecule Type: Synthetic DNA

Sequence

CCCTCCACCG AGGACCXTGG TCAACTTACT CCC 32

SEQ ID NO: 21

Length: 33

Sequence Type: Nucleic acid

Strandness: Single strand

Topology: Linear

Molecule Type: Synthetic DNA

Sequence

GGCGCCCTGG TCGXTCGGGG TCGTGTGCGC ACAG 33

SEQ ID NO: 22

Length: 32

Sequence Type: Nucleic acid

Strandness: Single strand

Topology: Linear

Molecule Type: Synthetic DNA

Sequence

GTGCAGTGGA TGAACCXGGC TGATAGCGTT CGC 32

SEQ ID NO: 23

Length: 31

Sequence Type: Nucleic acid

Strandness: Single strand

Topology: Linear

Molecule Type: Synthetic DNA

Sequence

TGATAGCGTT CGCTTCGCXG GGGTAACCAC GT 31

SEQ ID NO: 24

Length: 29

Sequence Type: Nucleic acid

Strandness: Single strand

Topology: Linear

Molecule Type: Synthetic DNA

Sequence

ACGCATTATG TGCXCTGAAA GCGACGCTGC 29

SEQ ID NO: 25

Length: 31

Sequence Type: Nucleic acid

Strandness: Single strand

Topology: Linear

Molecule Type: Synthetic DNA

Sequence

TGGCGGGTGG CTGCGGAGGX AGTACGTGGA GG 31

SEQ ID NO: 26

Length: 33

Sequence Type: Nucleic acid

Strandness: Single strand

Topology: Linear

Molecule Type: Synthetic DNA

Sequence

GGTGGGGGAT TTCCXACTAC GTGACGGGCA TGAC 33

SEQ ID NO: 27

Length: 32

Sequence Type: Nucleic acid

Strandness: Single strand

Topology: Linear

Molecule Type: Synthetic DNA

Sequence

GAATTCTTCA CGGAGTTGGX ATGGGGTACG GCT 32

SEQ ID NO: 28

Length: 31

Sequence Type: Nucleic acid

Strandness: Single strand

Topology: Linear

Molecule Type: Synthetic DNA

Sequence

CCAGCTCTTC AGXCTAGTCA GTTGTCTGCA CC 31

SEQ ID NO: 29

Length: 30

Sequence Type: Nucleic acid

Strandness: Single strand

Topology: Linear

Molecule Type: Synthetic DNA

Sequence

TGGCGGCAGG AGATGGXGCG GAAACATCAC C 30

SEQ ID NO: 30

Length: 31

Sequence Type: Nucleic acid

Strandness: Single strand

Topology: Linear

Molecule Type: Synthetic DNA

Sequence

AGTCAGAGAA CAAGGXTAGT AATCCTAGAC TC 31

SEQ ID NO: 31

Length: 22

Sequence Type: Nucleic acid

Strandness: Single strand

Topology: Linear

Molecule Type: Synthetic DNA

Sequence

ACATCCTGGC GGGTTATGGA GC 22

SEQ ID NO: 32

Length: 22

Sequence Type: Nucleic acid

Strandness: Single strand

Topology: Linear

Molecule Type: Synthetic DNA

Sequence

TGCCCTCCAC CGAGGACCTG GT 22

SEQ ID NO: 33

Length: 19

Sequence Type: Nucleic acid

Strandness: Single strand

Topology: Linear

Molecule Type: Synthetic DNA

Sequence

TGATAGCGTT CGCTTCGCG 19

SEQ ID NO: 34

Length: 22

Sequence Type: Nucleic acid

Strandness: Single strand

Topology: Linear

Molecule Type: Synthetic DNA

Sequence

CGCTTTCAGG CACATAATGC GT 22

SEQ ID NO: 35

Length: 21

Sequence Type: Nucleic acid

Strandness: Single strand

Topology: Linear

Molecule Type: Synthetic DNA

Sequence

GCACTATGTG CCTGAAAGCG A 21

SEQ ID NO: 36

Length: 22

Sequence Type: Nucleic acid

Strandness: Single strand

Topology: Linear

Molecule Type: Synthetic DNA

Sequence

TGGCTCAGGG ATGTTTGGGA CT 22

SEQ ID NO: 37

Length: 22

Sequence Type: Nucleic acid

Strandness: Single strand

Topology: Linear

Molecule Type: Synthetic DNA

Sequence

AGTCCCAAAC ATCCCTGAGC CA 22

SEQ ID NO: 38

Length: 20

Sequence Type: Nucleic acid

Strandness: Single strand

Topology: Linear

Molecule Type: Synthetic DNA

Sequence

TGGAGCCAGG TTTTGAAGTC 20

SEQ ID NO: 39

Length: 20

Sequence Type: Nucleic acid

Strandness: Single strand

Topology: Linear

Molecule Type: Synthetic DNA

Sequence

TTGTACCCTC GTTGGCATGA 20

SEQ ID NO: 40

Length: 19

Sequence Type: Nucleic acid

Strandness: Single strand

Topology: Linear

Molecule Type: Synthetic DNA

Sequence

GGCCTGTGGT GTATGCGTT 19

SEQ ID NO: 41

Length: 22

Sequence Type: Nucleic acid

Strandness: Single strand

Topology: Linear

Molecule Type: Synthetic DNA

Sequence

AACGCATACA CCACAGGCCC CT 22

SEQ ID NO: 42

Length: 23

Sequence Type: Nucleic acid

Strandness: Single strand

Topology: Linear

Molecule Type: Synthetic DNA

Sequence

GTGGCTGCGG AGGAGTACGT GGA 23

SEQ ID NO: 43

Length: 22

Sequence Type: Nucleic acid

Strandness: Single strand

Topology: Linear

Molecule Type: Synthetic DNA

Sequence

TCCACGTACT CCTCCGCAGC CA 22

SEQ ID NO: 44

Length: 21

Sequence Type: Nucleic acid

Strandness: Single strand

Topology: Linear

Molecule Type: Synthetic DNA

Sequence

GAACGTGACC TCATCCCGCA G 21

SEQ ID NO: 45

Length: 23

Sequence Type: Nucleic acid

Strandness: Single strand

Topology: Linear

Molecule Type: Synthetic DNA

Sequence

GTGAGCACCG CCACGTCCGG TTC 23

SEQ ID NO: 46

Length: 23

Sequence Type: Nucleic acid

Strandness: Single strand

Topology: Linear

Molecule Type: Synthetic DNA

Sequence

GTGGCGGTGC TCACTTCCAT GCT 23

SEQ ID NO: 47

Length: 20

Sequence Type: Nucleic acid

Strandness: Single strand

Topology: Linear

Molecule Type: Synthetic DNA

Sequence

ATGCTCACCG ACCCCACCCA 20

SEQ ID NO: 48

Length: 20

Sequence Type: Nucleic acid

Strandness: Single strand

Topology: Linear

Molecule Type: Synthetic DNA

Sequence

CATGACTCCC CAGACGCTGA 20

SEQ ID NO: 49

Length: 20

Sequence Type: Nucleic acid

Strandness: Single strand

Topology: Linear

Molecule Type: Synthetic DNA

Sequence

TACTTCCCTC TCATCCTCTT 20

SEQ ID NO: 50

Length: 20

Sequence Type: Nucleic acid

Strandness: Single strand

Topology: Linear

Molecule Type: Synthetic DNA

Sequence

GTTTTCCGCA GGATCTCTGC 20

SEQ ID NO: 51

Length: 20

Sequence Type: Nucleic acid

Strandness: Single strand

Topology: Linear

Molecule Type: Synthetic DNA

Sequence

CTGCACAGGT ACGCTCCGGC 20

SEQ ID NO: 52

Length: 20

Sequence Type: Nucleic acid

Strandness: Single strand

Topology: Linear

Molecule Type: Synthetic DNA

Sequence

CTCCTGCGGG ATGAGGTCAC 20

SEQ ID NO: 53

Length: 20

Sequence Type: Nucleic acid

Strandness: Single strand

Topology: Linear

Molecule Type: Synthetic DNA

Sequence

GGCGCAGACA ACTGACTAGC 20

SEQ ID NO: 54

Length: 20

Sequence Type: Nucleic acid

Strandness: Single strand

Topology: Linear

Molecule Type: Synthetic DNA

Sequence

CCGCCCATCT CCTGCCGCCA 20

SEQ ID NO: 55

Length: 30

Sequence Type: Nucleic acid

Strandness: Single strand

Topology: Linear

Molecule Type: Synthetic DNA

Sequence

CACAGCTCCC ATGTGAGCCC GAACCGGATG 30

SEQ ID NO: 56

Length: 22

Sequence Type: Nucleic acid

Strandness: Single strand

Topology: Linear

Molecule Type: Synthetic DNA

Sequence

ACATCCTGGC GGGTTATGGA GC 22

SEQ ID NO: 57

Length: 22

Sequence Type: Nucleic acid

Strandness: Single strand

Topology: Linear

Molecule Type: Synthetic DNA

Sequence

AGTCCCAAAC ATCCCTGAGC CA 22

SEQ ID NO: 58

Length: 21

Sequence Type: Nucleic acid

Strandness: Single strand

Topology: Linear

Molecule Type: Synthetic DNA

Sequence

TGCCCTCCAC CGAGGACCTG G 21

SEQ ID NO: 59

Length: 22

Sequence Type: Nucleic acid

Strandness: Single strand

Topology: Linear

Molecule Type: Synthetic DNA

Sequence

CGCTTTCAGG CACATAATGC GT 22

SEQ ID NO: 60

Length: 31

Sequence Type: Nucleic acid

Strandness: Single strand

Topology: Linear

Molecule Type: Synthetic DNA

Sequence

TGATAGCGTT CGCTTCGCGG GGTAACCACG T 31

## Claims

1. A method of detecting a blood borne non-A, non-B hepatitis-specific gene based on DNA-DNA, DNA-RNA or RNA-RNA hybridization, characterized by using, as a probe, a cDNA inserted into one of the phage clones shown in Table 1, or a part thereof.

2. A method of detecting a blood borne non-A, non-B hepatitis-specific gene according to claim 1, wherein the probe is a DNA comprising one of the nucleotide sequences shown in SEQ ID NOs: 1 to 18, or a part thereof.

3. A method of detecting a blood borne non-A, non-B hepatitis-specific gene according to claim 1 or 2, wherein the probe comprises a part having a high mutual homology to the nucleotide sequences shown in SEQ ID NOs: 1 to 18.

4. A method of detecting a blood borne non-A, non-B hepatitis-specific gene according any one of claims 1 to 3, using a probe comprising one of the nucleotide sequences belonging to a region of an at least 25 mer length having at least an 80% homology between any of two arbitrarily selected nucleotide sequences, in the region wherein at least two nucleotide sequences are shown in parallel in Figs. 1 and 2.

5. A method of detecting blood borne non-A, non-B hepatitis-specific gene based on DNA-DNA, DNA-RNA or RNA-RNA hybridization, characterized by using a probe comprising a nucleotide sequence containing 5-fluorouracil or inosine replacement, wherein the nucleotide sequence belongs to a region of an at least 25 mer length having at least an 80% homology between any of two arbitrarily selected nucleotide sequences when one of nucleotides which are different at a corresponding site between nucleotide sequences is replaced by 5-fluorouracil or inosine, in the region wherein at least two nucleotide sequences are shown in parallel in Figs. 1 and 2.

6. A method of detecting a blood borne non-A, non-B hepatitis-specific gene based on a nucleic acid amplification method, characterized by using, as a primer, a part of a nucleotide sequence of a cDNA inserted into one of the phage clones shown in Table 1.

7. A method of detecting a blood borne non-A, non-B hepatitis-specific gene according to claim 6, characterized in that the primer comprises one of the nucleotide sequences shown in SEQ ID NOs: 1 to 18, or a part thereof.

8. A method of detecting a blood borne non-A, non-B hepatitis-specific gene according to claims 6 or 7, characterized in that the primer comprises a part having a high mutual homology to the nucleotide sequences shown in SEQ ID NOs: 1 to 18.

9. A method of detecting a blood borne non-A, non-B hepatitis-specific gene according to any one of claims 6 to 8, using a probe comprising one of the nucleotide sequences belonging to a region of at least a 15 mer length having at least an 80% homology between any of two arbitrarily selected nucleotide sequences, in the region wherein at least two nucleotide sequences are shown in parallel in Figs. 1 and 2.

10. A method of detecting a blood borne hepatitis-specific gene, based on nucleic acid amplification, characterized by using a primer comprising a nucleotide sequence containing 5-fluorouracil or inosine replacement, wherein the nucleotide sequence belongs to a region of at least a 15 mer length having at least an 80% homology between any of two arbitrarily selected nucleotide sequences when one of nucleotides which are different at a corresponding site in nucleotide sequences is replaced by 5-fluorouracil or inosine, in the region wherein at least two nucleotide sequences are shown in Figs. 1 and 2.

11. An agent for detecting a blood borne non-A, non-B hepatitis-specific gene, comprising a probe according to any one of claims 1 to 5, and/or primers according to any one of claims 6 to 10.

12. A DNA sequence encoding a non-A, non-B hepatitis-specific antigenic protein selected from the group consisting of
    (a) the DNA sequence inserted into one of the phage clones shown in Table 1 or a part thereof;
    (b) the DNA sequence according to any one of the sequences shown in SEQ ID Nos. 1 to 18 or a part thereof,
    (c) a DNA sequence hybridizing to the DNA sequence of (a) and (b) and encoding a protein with a highly specific serological reaction to non-A, non-B hepatitis,
    (d) a DNA sequence which is degenerate with respect to a DNA sequence according to (a), (b) or (c).

# Fig.1(1)

```
              10         20         30         40         50         60         70         80         90        100
#2256 CGGCTTTCGT GGGCGCCGGC ATAGCCGGCG CGGCTGTTGG CAGCATAGGC CTTGGGAAGG TGCTTGTGGA CATCCTGGCG GGTTATGGAG CAGGGGTGGC

             110        120        130        140        150        160        170        180        190        200
#2256 AGGCGCACTC GTGGTCTTTA AGGTTATGAG TGGCGACATG CCCTCCACCG AGGACCTGGT CAACTTACTC CCTGCCATCC TTTCCCCTGG CGCCCTGGTG

             210        220        230        240        250        260        270        280        290        300
#2256 GTCGGCGTCG TGTGCGCACA GATACTGCGT CGACATGTCG GCCCAGGGGA GGGAGCTGTG CAGTGGATGA ACCGGCTGAT AGCGTTCGCT TCGCGGGGTA
#2207                                                                                   ------ ---------- --------A-

             310        320        330        340        350        360        370        380        390        400
#2256 ACCACGTCTC CCCCACGCAT TATGTGCCTG AAAGCGACGC TGCGAGTCGT GTCACCCAGA TCCTCTCCAG CCTTACCATC ACTCAGCTGT TGAAGAGGCT
#2207 ---------- ---------- ---------- ---------- ---AGCG--- ---------- ---------- ---------- ---------- ----------

             410        420        430        440        450        460        470        480        490        500
#2256 CCACCAGTGG ATTAATGAGG ACTGCTCCAC GCCATGCTCC GGCACGTGGC TCAGGGATGT TTGGGACTGG ATATGCACGG TGTTGGCTGA CTTCAAGACC
#2207 ---------- --C------- ---------- ---------- --TT------ -T-------- ---------- ---------- -----A---- ------A---

             510        520        530        540        550        560        570        580        590        600
#2256 TGGCTCCAGT CCAAGCTCCT GCCGCGGTTA CCGGGGGTCC CTTTCGTCTC ATGTCAGCGT GGGTACAAGG GAGTTTGGCC GGGAGATGGC ATCATGCACA
#2207 ---------- ---------- ------A--G -----A---- ---------- ---C--A--A ---------- ----C----- ---------T G-------A-

             610        620        630        640        650        660        670        680        690        700
#2256 CCACCTGCCC ATGCGGAGCC CAAATCACCG GACATGTCAA AAACGGGTCC ATGAGGATCG CCGGGCCTAA AACCTGCAGC AACACGTGGC ACGGAACGTT
#2207 ---------- ---T-----A G-G----GT- -G-------- ---T--C--- ---------- TT-------G ------T--- ---------- ----------
#2244                                - -G-------- ---T--C--- ---------- TT-------G ------T--- ---------- ----------
```

Fig.1(2)

```
                710        720        730        740        750        760        770        780        790        800
#2256   CCCCATTAAC GCATACACCA CAGGCCCCTG CACACCCTCT CCGGCGCCAA ACTACTCCAG GGCGTTGTGG CGGGTGGCTG CGGAGGAGTA CGTGGAGGTC
#2207   ---T--C--- --G------- ---------- ---------C --------C- -------T-- ---------- ---------- -T-------- ----------
#2244   ---T--C--- --G------- ---------- ---------C --------C- -------T-- ---------- ---------- -T-------- ----------

                810        820        830        840        850        860        870        880
#2256   ACGCGGGTGG GGGATTTCCA CTACGTGACG GGCATGACCA CTGACAATGT AAAATGCCCA TGCCAGGTTC CGGCCCCCGA ATTC
#2207   ---------- ---------- ---------- ---------- -------C-- --G------- ---------- ---------- ----
#2244   ---------- ---------- ---------- ---------- -------C-- --G------- ---------- ---------- ----
```

# Fig.2(1)

```
               10         20         30         40         50         60         70         80         90        100
#2232  GAATTCTTCA CAGAGTTGGA TGGGGTGCGG CTGCACAGGT ACGCTCCGGC GTGCAAACCT CTCCTGCGGG ATGAGGTCAC GTTCCAGGTC GGGCTCAACC
#2230  ---------- ---------- ---------- ---------- ---------- ---------- ---------- ---------- ---------- ----------
#2206  ---------- ---------- C--------- ---------- ---------- -----G---- ---------- ---------- A--------- ----------
#2258  ---------- ---------- C--------- ---------- ---------- -----G---- ---------- ---------- A--------- ----------
#2248  ---------- ---------- ---------- T--------- ---------- ---------- -----A---- ---------- A--T------ ----------
#2220  ---------- -----C---- ---------- T--------- ---------- ------G--- -----A---- ---------- A--T------ ----------
#2216  ---------- ---------- ---------- T--------- ---------- TGCA--G--- -----A---- ---------- A--T------ ----------
#2246  ---------- -G-------- ------A--- ---------- ---------- ------G--A -----A---- ---------- A--------- ----------
#2211  ---------- -G--A----- ---------- --A------- ---------- ------G--- -----A---- ---------- A--------- ----------

              110        120        130        140        150        160        170        180        190        200
#2232  AATACCCGGT TGGATCACAG CTCCCATGCG AGCCCGAACC GGATGTGGCG GTGCTCACTT CCATGCTCAC CGACCCCACC CACATTACAG CAGAAGCGGC
#2230  ---------- ---------- ---------- ---------- ---------- ---------- ---------- ---------- ---------- ----------
#2206  ---------- ---G----C- --------T- ---------- ------AA-A ---G------ ---------- -------T-- ---------- -G---A-T--
#2258  ---------- ---G------ --------T- ---------- ------AA-A ---G------ ---------- -------T-- ---------- -G---A-T--
#2248  ---T------ ---G------ --------T- ---------- ------AA-A ---A----C- ---------- -------T-- ---------- ----GA----
#2220  ---T------ ---G------ -----G--T- ---------- ------AA-- ---A------ ---------- -------T-- ---------- ----GA----
#2216  ---T------ ---G------ --------T- ---------- ------AA-A ---A----C- ---------- -------T-- ---------- ----G-----
#2246  ---TT--A-- ---------- --------T- -------G-- ------A--- ---------- ---------- -------T-- ---------- ----GA----
#2211  ---T------ ---G------ --------T- -A-------- ------AAT- ---G----C- -T-------- -------T-- --T------- ----GA----

              210        220        230        240        250        260        270        280        290        300
#2232  TAGGCGCAGG CTGGCCAGAG GGTCTCCTCC TTCCTTGGCC AGCTCTTCAG CTAGTCAGTT GTCTGCGCCC TCCTTGAAGG CGACATGCAC TACCCATCAT
#2230  ---------- ---------- ---------- ---------- ---------- ---------- ---------- ---------- ---------- ----------
#2206  C-----T--- T-------G- --AG---C-- ---------- ---------- ---------- ------A--T ---------- -------T-- ----------
#2258  C-----T--- T-------G- --AG---C-- ---------- ---------- ---------- ------A--T ---------- -------T-- ----------
#2248  -G----T--- ---------- -------C-- ---------- --------G- ---------- ---------T ---------- ---------- -----G----
#2220  -G----T--- ---------- -------C-- ---------- --------G- ---------- ---------- ---------- -A-------- -----G----
#2216  -G----T--- ---------- -------C-- ---------- --------G- ---------- ---------- *---*----- *--------- -----G----
#2246  --A---T--- --------G- ----C--C-- C--------- ---------- ---------- ---------- --T------- ---------- -----C----
#2211  --A---T--- ---------- -------C-- ---T------ ---------- ---------- ---------- ---------- ---------- C----G----
```

# Fig.2(2)

```
             310        320        330        340        350        360        370        380        390        400
#2232  GACTCCCCAG ACGCTGACCT CATCGAGGCC AACCTCCTGT GGCGGCAGGA GATGGGCGGA AACATCACCC GCGTGGAGTC AGAGAATAAG GTAGTAATTC
#2230  ---------- ---------- ---------- ---------- ---------- ---------- ---------- ---------- ---------- ----------
#2206  -----T---- -------T-- ---------- -----T--A- ---------- ---------G ---------- -T-------- ---------- --------C-
#2258  -----T---- -------T-- ---------- -----T--A- ---------- ---------G ---------- -T-------- ---------- --------C-
#2248  ---------- ---------- ---------- --T------- ---------- ------A--G ---------- ---------- ------C--- --------C-
#2220  ---------- ---------- ---------- --T------- ---------- ------A--G ---------- ---------- ------C--- --------C-
#2216  ---------- ---------- ---------- --T------- ---------- ------A--G ---------- ---------- ------C--- ---C----C-
#2246  --------G- ---------- ---------- ---------- ---------- ------A--- ---------- ---------- ---------- ----------
#2211  --------G- ---------- G--A------ ---------- ---------- ---------G ---------- -T-------- ---------- -----G---T

             410        420        430        440        450        460        470        480        490        500
#2232  TACAGTGTTT TGAAGGGGTT GGAGGGGAAG AGGATGACAG GGAACTATGG GTTGGGGGAG ACATGGTGGG GAAAAGGAGG AGATTGGGGG GACGGATGGG
#2230  ---------- ---------- ---------- ---------- ---------- ---------- ---------- ---------- ---------- ----------
#2206  ---------- ---C------ --------G- ---------- ---------- --------G- ---------- --G------- ---------C -G-----A--
#2258  ---------- ---C------ --------G- ---------- ---------- --------G- ---------- --G------- ---------C -G--------
#2248  ---------- ---C-----C --------G- ---------- ---GA----T --------G- -------A-- ----T-T--- -A-------C ----AT----
#2220  ---------- ---C-----C --------G- ---------- ---GA----T --------G- -------A-- ----T-T--- -A-------C ----AT----
#2216  ---------- ---C-----C --------G- ---------- ---GA----T --------CC --C-
#2246  ---A------ ---------C --------G- ---------- ------G--- --------GG --A------- -----G---- -
#2211  -G-------- ---------- --G-T---G- ---------- ---------- --A-----G- -

             510        520        530        540        550        560        570        580        590        600
#2232  CATATGGGCA CGTCCGGACT ACAACCCACC ATTACTACAG TCCTGGAAGG ACCCGGACTA CGTCCCTCCG GTGGTGCACG GGTGCCCATT GCCACCTGCC
#2230  ---------- ---------- ---------- ---------- ---------- ---------- ---------- ---------- ---------- -------
#2206  TG-------G --C------- -------G-- -C-GA--G-A --T------- ----A----- ------A--- -----A---- ----T----- ----------
#2258  ---------G --C------
#2248  ---------G --C------- ---------- -C-G------ --T---
#2220  ---------G --C------- ------
```

# INTERNATIONAL SEARCH REPORT

International Application No PCT/JP91/01452

**I. CLASSIFICATION OF SUBJECT MATTER** (if several classification symbols apply, indicate all) [6]

According to International Patent Classification (IPC) or to both National Classification and IPC

Int. $Cl^5$ C12Q1/70, 1/68//C12N15/51, 15/11,
(C12Q1/70, C12R1:92)

**II. FIELDS SEARCHED**

Minimum Documentation Searched [7]

| Classification System | Classification Symbols |
|---|---|
| IPC | C12Q1/70, 1/68, C12Q15/51, 15/11 |

Documentation Searched other than Minimum Documentation to the Extent that such Documents are Included in the Fields Searched [8]

JICST data base

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, A, 64-2576 (Mitsubishi Kasei Corp.), January 6, 1989 (06. 01. 89) & US, A, 5,032,511 & EP, B, 293,274 | 1-11 |
| A | Experimental Medicine, Vol. 7, No. 2, 1989, Terukatsu Arima "Cloning of Hemo-propagation non-A non-B Hepatitis Virus" p. 196-201 | 1-11 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| January 21, 1992 (21. 01. 92) | February 12, 1992 (12. 02. 92) |
| **International Searching Authority** | **Signature of Authorized Officer** |
| Japanese Patent Office | |